# EUROPEAN PATENT SPECIFICATION

(11) **EP 0 634 142 B1**
(45) Date of publication and mention of the grant of the patent: **11.03.1998**
(21) Application number: 94304278.8
(22) Date of filing: 14.06.1994
(51) Int. Cl.: A61B 17/04

(54) **Biocompatible suture knot clip**
Bioverträgliche Klammer zum Verknoten eines Nähfaden
Pince biocompatible pour faire les noeuds d'une suture

(30) Priority: 15.06.1993 US 76879
(43) Date of publication of application: 18.01.1995
(73) Proprietor: ETHICON INC., Somerville, New Jersey 08876-0151 (US)
(72) Inventor: Zwaskis, William J., Fanwood, New Jersey 07032 (US)
(74) Representative: Mercer, Christopher Paul

(56) References cited:
- FR-A- 1 534 916
- FR-A- 2 682 867
- US-A- 4 750 492

## Description

### FIELD OF INVENTION

The present invention relates to a surgical clip. More particularly, it relates to a clip suitably adapted to replace a suture knot during endoscopic surgery.

### BACKGROUND OF THE INVENTION

One of the truly great advances in recent years to reduce the invasiveness of surgical procedures is endoscopic surgery. Endoscopic surgery involves the use of an endoscope which is an instrument permitting the visual inspection and magnification of any cavity of the body. The endoscope is inserted through a cannula after puncture through the wall of the body cavity with a trocar, which is a sharp-pointed instrument. The surgeon can then perform diagnostic and therapeutic procedures at the surgical site with the aid of specialized instrumentation designed to fit through additional cannulas providing small diameter openings into the desired body cavity as may be required.

An age-old procedure which surgeons are required to perform to repair or reconstruct traumatized bodily tissue is suturing. Fortunately, medical instruments have been recently designed to allow a surgeon to manipulate a suture, or suture and needle combination, through the small diameter opening of a cannula. However, the ability to tie an appropriately placed suture knot had become troublesome and problematical.

Therefore, in response to this problem, surgeons have sought alternatives to conventional knot-tying techniques which would be suitable during endoscopic surgery. These alternatives include the use of hemostatic clips, which are designed to ligate blood vessels and other tubular members, to replace suture knots. Such hemostatic clips are described, for example, in U.S. Pat Nos. 4,418,694 and 4,476,865. These clips can be readily applied with a clip applier which is designed to function through the small opening of a cannula. Unfortunately, the force required to displace these clips from a suture is too low to provide a secure knot. As a result, hemostatic clips of the type shown in the art are unsuitable for use as endoscopic knot clips.

From FR-A-2682867, a surgical suture clip, having the features as defined in the preamble of claim 1, is known.

In view of the deficiencies of the prior art for creating a useful alternative to tying a suture knot, what is desired within the medical community is a device suitable for application using endoscopic techniques which can successfully replace the suture knot. More specifically, what is needed is a clip particularly adapted for replacing a suture knot during endoscopic surgery, and which exhibits adequate clamping force to function effectively.

Summary of the invention.

The present invention provides a sterile surgical suture clip comprising an insertion member having means for retaining one or more sutures, and a locking notch. The locking notch is engageable with an engaging means located on a receiving member. The receiving member has a passage provided with a first opening and a second opening, and the receiving member is adapted to allow the insertion member to be slid within the passage of the receiving member. Once slid within the passage, the insertion member is locked in the receiving member by the interaction of the engaging means with the locking notch. In such a locked position the suture is locked. The sterile surgical suture clip is characterised in that the insertion member is provided with a retaining notch such that the insertion member can be retained in the receiving member by the interaction of the engaging means with the retaining notch, whereby in such a retained position the one or more sutures may be placed in the means for retaining one or more sutures.

In a further development of the sterile surgical suture clip, means for retaining one or more sutures comprises a channel and opposite guides into which one or more sutures may be placed.

An alternative form of the present invention comprises a sterile surgical suture clip wherein the insertion member and receiving member have a circular cross section.

### BRIEF DESCRIPTION OF THE FIGURES

The present invention will be described in great detail in conjunction with the accompanying figures.

FIG. 1 is an enlarged perspective view of a clip in accordance with one embodiment of the present invention.

FIG. 2 an enlarged view of the receiving member of the suture clip illustrated in FIG. 1.

FIG. 3 an enlarged perspective view of the insertion member of the suture clip illustrated in FIG. 1.

FIG. 4 a cross sectional view of FIG. 1 taken along line 4-4.

FIG. 5 is a perspective view of the suture clip clipped about two sutures.

FIG. 6 is a cross-sectional view along line 6-6 of FIG. 5.

FIG. 7 illustrates an alternate embodiment of the invention wherein the insertion member 4 and lumen 10 of the receiving member 6 have a circular cross-section.

### DETAILED DESCRIPTION OF THE INVENTION

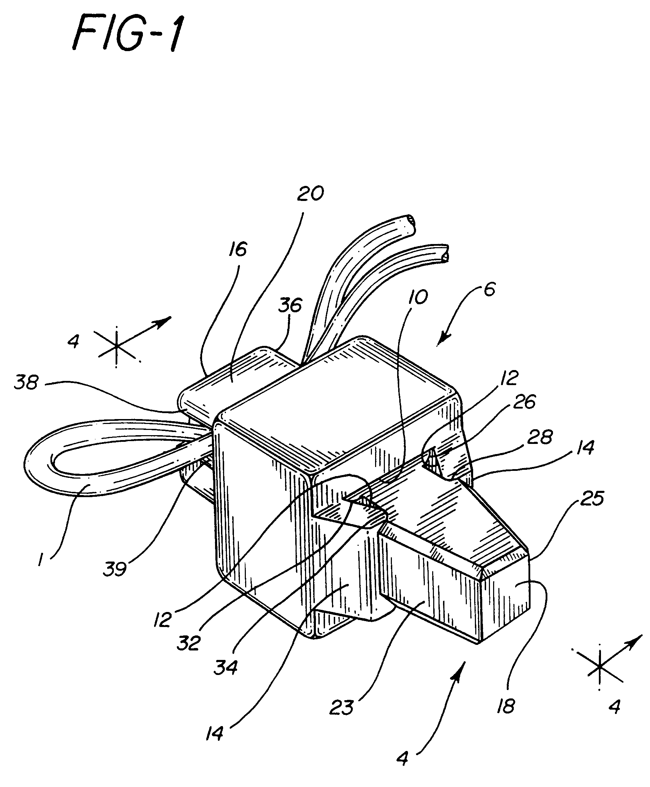

Referring to FIG. 1 there is shown a surgical suture clip of the present invention. The clip 2 has a insertion member 4 and a receiving member 6.

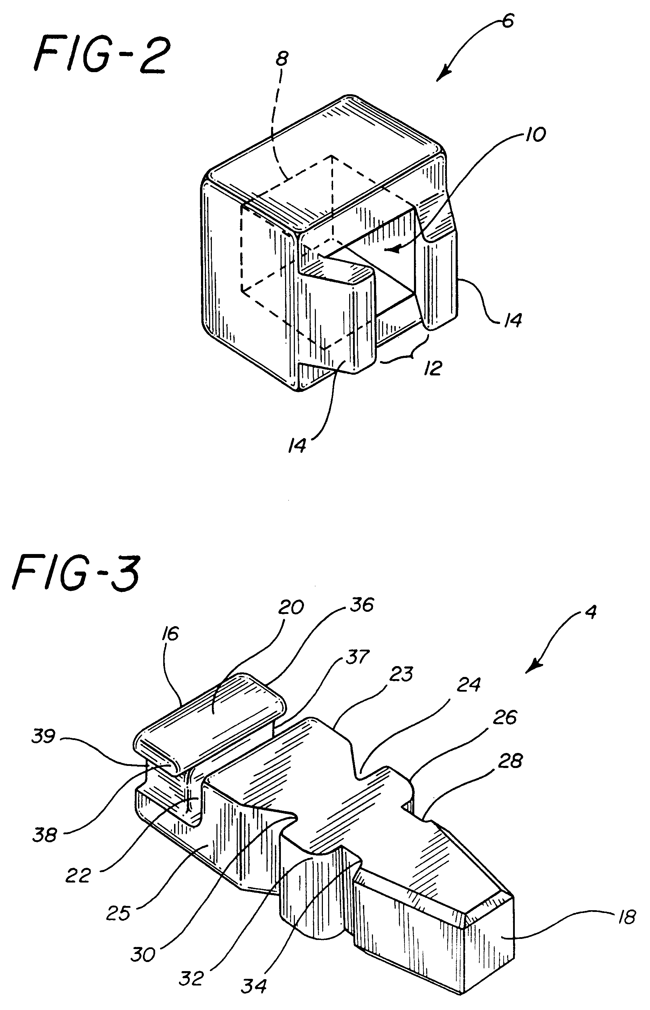

The receiving member 6, as illustrated in FIG.2, has a first opening 8, a passage 10, a second opening 12, and engaging means 14.

The insertion member 4, as illustrated in FIG. 3, has a proximal end 16, a distal end 18, a dorsal surface 20, a first side 23 and a second side 25 (opposed to said first side). Near the proximal end 16 of the insertion member 4 on the first side is located first surface 36. In a preferred embodiment of the invention substantially parallel to first surface 36 and recessed therein is guide 37. Guide 37 forms a recessed channel in first surface 36 in which one or more sutures may be placed. Opposed to the first surface 36 on the second side 25 of the insertion member is second surface 38. In a preferred embodiment of the invention substantially parallel to the second surface 38 and recessed therein is guide 39. Guide 39 forms a recessed channel in second surface 38 in which one or more sutures may be placed. Located toward the distal end of the insertion member from the first surface 36 is channel 22. Channel 22 is oriented transversely across the insertion member 4 (from said first side 23 to said second side 25) and is of sufficient width and depth to accommodate one or more sutures. Spaced toward the distal end from the channel 22 on the first side 23 of the insertion member 4 is a locking notch 24. The insertion member may be beveled from the distal end to the locking notch 24 to facilitate the insertion of the insertion member 4 into receiving member 6. In a preferred embodiment, as is shown in FIG. 1-7, adjacent to the locking notch toward the distal end of the insertion member is shoulder 26. Adjacent to said shoulder toward the distal end is retaining notch 28. The insertion member may be beveled from the distal end to the retaining notch 28 to facilitate the insertion of the insertion member 4 into receiving member 6. In a preferred embodiment of this invention there may also be disposed on the second side surface 25 in substantially a mirror image placement to the locking notch 24, shoulder 26 and retaining notch 28 of the first side are locking notch 30, shoulder 32 and retaining notch 34. This preferred embodiment is illustrated in FIG. 3.

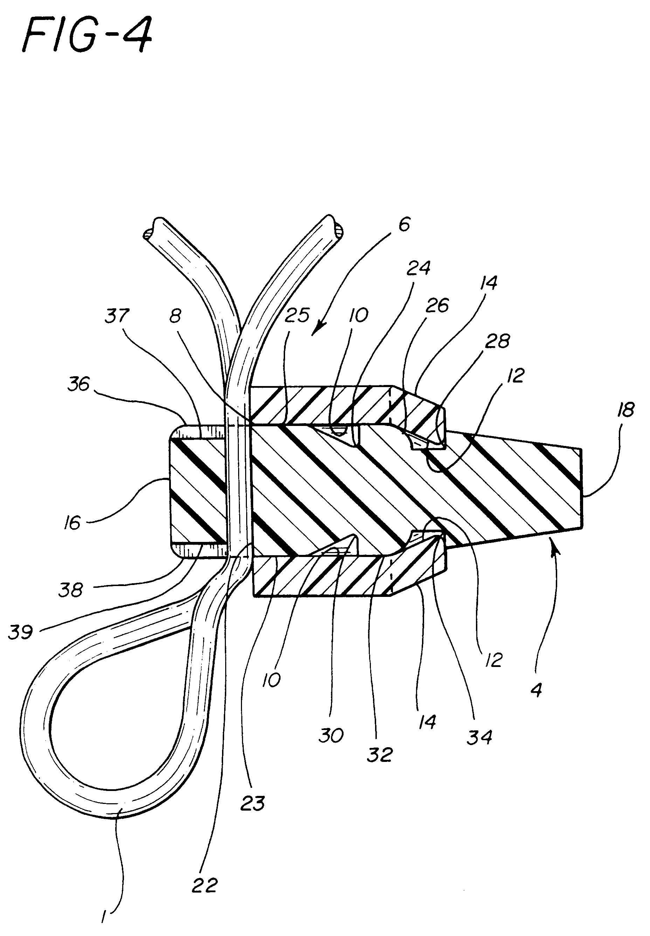

The clip is assembled by placing the distal end 18 of the insertion member 4 through the first opening 8 of the receiving member 6. The insertion member 4 and receiving member 6 are adapted to allow the insertion member to slide within passage 10 of the receiving member. Advancing the distal end of the insertion member 4 through the second opening 12 thereby deforming engaging means 14 until the retaining notch 28 allows the engaging means to snap back to substantially its original shape. This assembly is illustrated in FIG. 1 and in cross-section in FIG.4. The clip as shown in FIG. 1 and FIG.4 is a one piece unit.

In use one or more sutures can be placed within channel 22, as is illustrated in FIG 1. The insertion member 4 is then advanced. As the insertion member 4 slides within the passage 10 of the receiving member 6 shoulder 26 contacts engaging means 14 and deforms engaging means 14. The engaging means 14 after passing over shoulder 26 snap into locking notch 24. Simultaneously channel 22 passes into the passage 10 of the receiving member 6. The suture 1 is then compressively or frictionally engaged between the passage 10 and the first and second surfaces respectively 36 and 38 of the insertion member 4. In a preferred embodiment of the invention one or more opposed guides such as first guide 37 and second guide 39 are provided to engage said suture to avoid shearing said suture between channel 22 and opening 8.

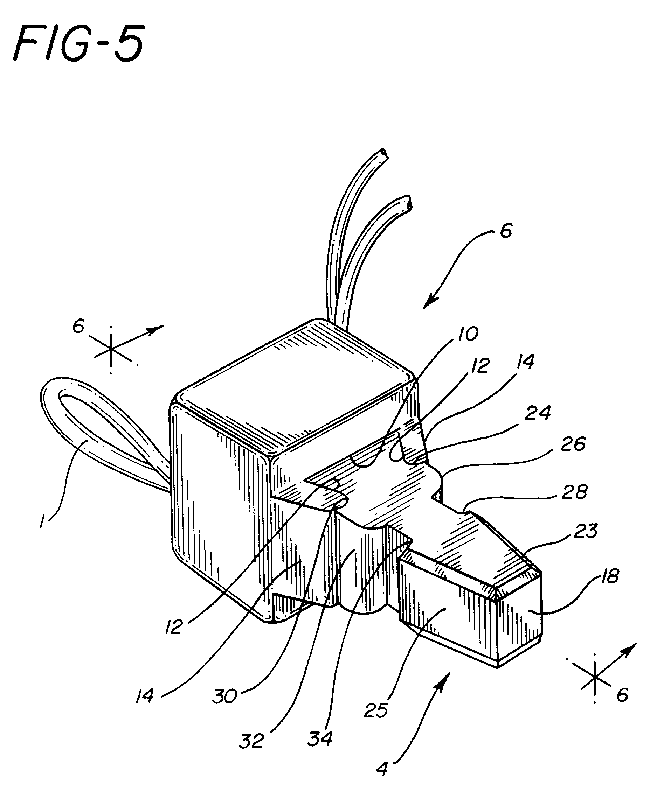

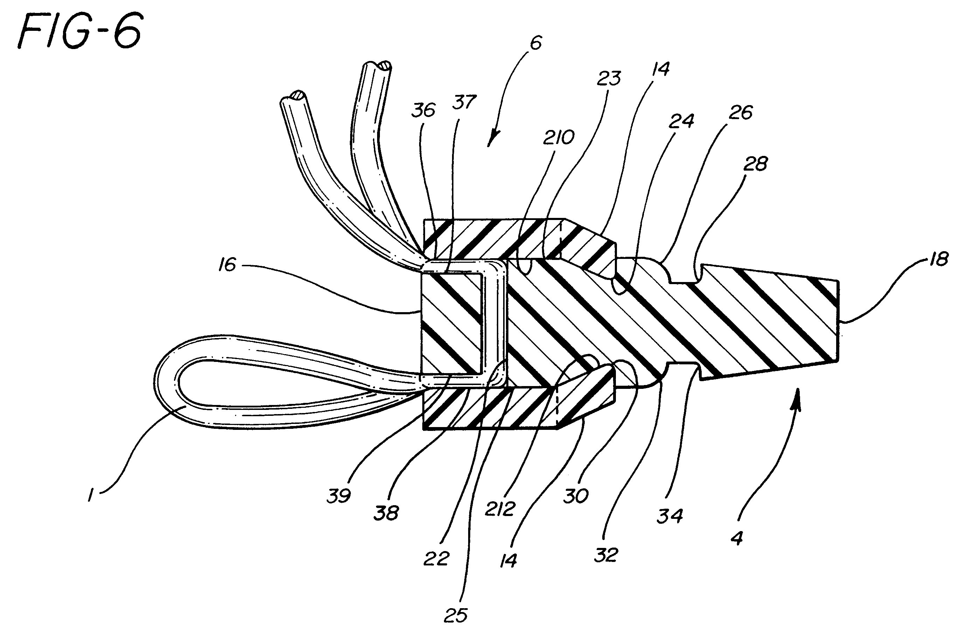

The suture 1 secured within the suture clip is illustrated in FIG. 5. A cross-sectional view of FIG. 5 illustrating the frictional engagement of the suture between the passage 10 and the first and second guides respectively 37 and 39 is provided in FIG. 6. The sizes of the receiving member and insertion member, as well as, the dimensions of the channel 22, the passage 10 and the width of the insertion member from the first guide to the second guide may be adjusted or sized to provide an appropriate frictional or compressive force to retain the suture 1.

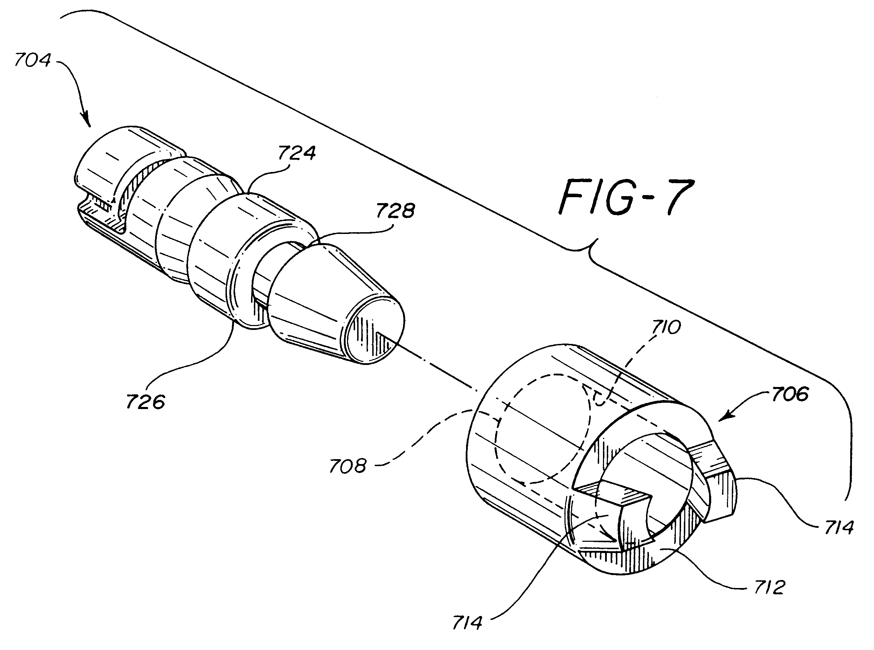

In an alternate embodiment of the invention the cross-section of the suture clip may be cylindrical as is illustrated in FIG. 7. In this embodiment the retaining notch 728 and the locking notch 724 would form annular grooves around the cylindrical cross-section of insertion member 704. Shoulder 726 in this embodiment would be an annular ring. Similarly the receiving member 706 would also be configured to have a circular cross-section as well as a circular first opening 708, passage 710, second end 712 and engaging means 714 adapted to operate in substantially the same manner as the embodiment described above.

The clips of the present invention may be constructed in various sizes according to their intended function. Suture clips are usually less than 8 millimeters in length and 5 millimeters in width. The dimension of the clip may be substantially reduced for certain applications in microsurgery. Larger clips for special suture applications may be about double the size of a typical suture clip. The various sizes of the clip are preferably matched with individual appliers having jaws tailored to the size of the clip for best performance.

The clips of the invention can be made of any biocompatible material using conventional fabrication methods. The clips can be composed of various biocompatible metals, e.g. titanium and tantalum, and polymeric materials. Preferred bioabsorbable polymeric materials include homopolymers and copolymer of e-caprolactone, glycolide, lactide and para-dioxanone. Preferred non-absorbable polymers include nylons, polyesters and polypropylene. All these materials have been demonstrated to be biologically acceptable when used as sutures or other implantable medical devices.

The preferred means for fabricating clips from polymeric materials is to inject a suitable polymer melt into an appropriately designed mold at process conditions conventionally employed for such polymer systems. After the polymer melt cools, the molded polymer shaped in the mold to meet the design criteria of the clip can be readily released from the mold.

The clips are sterilized by any of the well known sterilization techniques and the technique selected will depend to a great extent on the material used to make the clip. Suitable sterilization techniques include heat or steam sterilization, radiation sterilization such as cobalt irradiation, electron beam and the like, ethylene oxide, and other sterilization techniques well known in the art.

Having now described the present invention and certain specific embodiments thereof, it will be readily apparent to those skilled in the art that many variations and modifications may be made to the present invention without departing from the scope thereof as defined in the appended claims.

## Claims

1. A sterile surgical suture clip (2) comprising an insertion member (4) having means for retaining one or more sutures (1), a locking notch (24) being engageable with an engaging means (14) located on a receiving member (6), said receiving member (6) having a passage (10) provided with a first opening (8) and a second opening (12), said receiving member (6) being adapted to allow the insertion member (4) to be slid within said passage (10) of the receiving member (6) and to be locked in said receiving member (6) by the interaction of said engaging means (14) with the locking notch (24) to lock the suture, and characterised in that the insertion member (4) is provided with a retaining notch (28) such that when the insertion member (4) is retained in said receiving member (6) by the interaction of said engaging means (14) with the retaining notch (28), the one or more sutures (1) may be placed in said means for retaining one or more sutures (1).

2. A sterile surgical suture clip (2) according to claim 1 wherein the means for retaining one or more sutures (1) comprises a channel (22) and opposite guides (37 & 39) into which one or more sutures (1) may be placed.

3. A sterile surgical suture clip (2) according to claim 1 or 2 wherein the insertion member (4) and receiving member (6) have a circular cross section.

4. A sterile surgical suture clip (2) according to any one of claims 1 to 3 wherein the one or more sutures (1) comprises two ends of one looped suture (1).

## Patentansprüche

1. Sterile chirurgische Nahtmaterialklammer (2) mit einem Einsatzelement (4) mit einer Einrichtung zum Halten von einem oder mehreren Nahtfäden (1) und mit einer Rasteinkerbung (24), die mit Eingriffsmitteln (14) an einem Aufnahmeelement (6) in Eingriff kommen kann, wobei das Aufnahmeelement (6) einen Durchgang (10) mit einer ersten Öffnung (8) und einer zweiten Öffnung (12) aufweist, und wobei das Aufnahmeelement (6) dafür vorgesehen ist, es zu ermöglichen, daß das Einsatzelement (4) im Durchgang (10) des Aufnahmeelementes (6) gleitet und im Aufnahmeelement (6) durch das Zusammenwirken der Eingriffsmittel (14) mit der Rasteinkerbung (24) zum Sperren des Nahtmateriales einrastet, dadurch **gekennzeichnet**, daß das Einsatzelement (4) derart mit einer Halteeinkerbung (28) versehen ist, daß, wenn das Einsatzelement (4) durch das Zusammenwirken der Eingriffsmittel (14) mit der Halteeinkerbung (28) im Aufnahmeelement (6) gehalten wird, der oder die Nahtfäden (1) in die Halteeinrichtung für ein oder mehrere Nahtfäden (1) eingelegt werden kann bzw. können.

2. Sterile chirurgische Nahtmaterialklammer (2) nach Anspruch 1, wobei die Einrichtung zum Halten von einem oder mehreren Nahtfäden (1) einen Kanal (22) und gegenüberliegende Führungen (37 & 39) umfaßt, in die ein oder mehrere Nahtfäden (1) eingelegt werden können.

3. Sterile chirurgische Nahtmaterialklammer (2) nach Anspruch 1 oder 2, wobei das Einsatzelement (4) und das Aufnahmeelement (6) einen kreisförmigen Querschnitt haben.

4. Sterile chirurgische Nahtmaterialklammer (2) nach einem der Ansprüche 1 bis 3, wobei der eine Nahtfaden oder die mehreren Nahtfäden (1) aus den beiden Enden eines schlaufenförmigen Nahtfadens gebildet werden.

## Revendications

1. Pince de suture chirurgicale stérile (2), comprenant un élément d'insertion (4) possédant des moyens de retenue d'une ou plusieurs sutures (1), une encoche de blocage (24) pouvant être enclenchée avec des moyens de clipsage (14) situés sur un élément récepteur (6), ledit élément récepteur (6) possédant un passage (10) muni d'une première ouverture (8) et d'une seconde ouverture (12), ledit élément récepteur (6) étant conçu pour permettre à l'élément d'insertion (4) de coulisser à l'intérieur dudit passage (10) de l'élément récepteur (6) et d'être bloqué dans ledit élément récepteur (6) par l'interaction desdits moyens de clipsage (14) avec l'encoche de blocage (24) afin de bloquer la suture, et caractérisé en ce que l'élément d'insertion (4) est muni d'une encoche de retenue (28) telle que l'élément d'insertion (4) est retenu dans ledit élément récepteur (6) par l'interaction desdits moyens de clipsage (14) avec l'encoche de retenue (28), la ou les sutures (1) pouvant être placées dans ledit moyen de retenue d'une ou plusieurs sutures (1).

2. Pince de suture chirurgicale stérile (2) selon la revendication 1, dans laquelle les moyens de retenue d'une ou plusieurs sutures (1) comprennent une rainure (22) et des glissières opposées (37 & 39) dans lesquelles une ou plusieurs sutures (1) peuvent être placées.

3. Pince de suture chirurgicale stérile (2) selon la revendication 1 ou la revendication 2, dans laquelle l'élément d'insertion (4) et l'élément récepteur (6) ont une section circulaire.

4. Pince de suture chirurgicale stérile (2) selon l'une quelconque des revendications 1 à 3, dans laquelle la ou les sutures (1) comprennent deux extrémités d'une suture en boucle (1).
